# EUROPEAN PATENT APPLICATION

(11) **EP 4 079 730 A1**
(43) Date of publication of application: **26.10.2022**
(21) Application number: 20901132.9
(22) Date of filing: 15.12.2020
(51) Int. Cl.: C07D 401/12, A61K 31/506, A61P 35/00

(54) **NOVEL DEUTERIUM-SUBSTITUTED PYRIMIDINE DERIVATIVE AND PHARMACEUTICAL COMPOSITION COMPRISING SAME**

(30) Priority: 16.12.2019 KR 20190167769
(71) Applicant: Oncobix Co., Ltd., Yongin-si, Gyeonggi-do 16950 (KR)
(72) Inventor: KIM, Sung-Eun, Yongin-si, Gyeonggi-do 16950 (KR); LEE, Sunho, Yongin-si, Gyeonggi-do 16950 (KR); RAJESH, Rengasamy, Yongin-si, Gyeonggi-do 16950 (KR); LEE, Yong Hyub, Yongin-si, Gyeonggi-do 16950 (KR); KONG, Yun Jeong, Yongin-si, Gyeonggi-do 16950 (KR)
(74) Representative: dompatent von Kreisler Selting Werner - Partnerschaft von Patent- und Rechtsanwälten mbB
(86) International application number: PCT/KR2020/018385
(87) International publication number: WO 2021/125758

(57) **Abstract**

The present invention provides: a novel deuterium-substituted pyrimidine derivative compound represented by Formula 1, or a pharmaceutically acceptable salt thereof; and a pharmaceutical composition for treating lung cancer, comprising the same. Particularly, the deuterium-substituted pyrimidine derivative compound represented by Formula 1 effectively inhibits the growth of ALK-mutated and EGFR-mutated cancer cells.

## Description

### [Technical Field]

The present invention relates to a novel deuterium-substituted pyrimidine derivative and a pharmaceutical composition comprising the same.

### [Background Art]

Non-small cell lung cancer (NSCLC) is a disease with very high prevalence and mortality of cancer-related diseases worldwide in recent years. Non-small cell lung cancer is caused by several factors, but is mainly caused by mutation, overexpression, or the like in tyrosine kinase or anaplastic lymphoma kinase gene, and anticancer drugs to treat them are being developed to target inhibition of the activity of these enzymes.

It is known that non-small cell lung cancer, which mainly occurs in East Asia including South Korea, often has epithelial growth factor receptor (EGFR) gene mutations; and activating mutations in the kinase region of an epidermal growth factor receptor (EGFR) have been found to be carcinogenic genes in some patients with non-small cell lung cancer, and gefitinib, erlotinib, and the like are used as therapeutic agents, i.e., low molecular weight epidermal growth factor receptor (EGFR) kinase inhibitors for treating them (Science 2004, 304: 1497-500; and New England Journal of Medicine 2004, 350: 2129-39). In patients with non-small cell lung cancer confirmed to have EGFR activity mutation, the use of gefitinib and erlotinib as therapeutic agents results in drug resistance in most patients within one year (Clinical Cancer Research 2013, 19: 2240-7). Among these resistance mechanisms, the T790M mutation rate of epidermal growth factor receptor was observed at up to 60%. Thus, a 3rd-generation EGFR inhibitor has been developed that targets the T790M mutant epidermal growth factor receptor (EGFR) in lung cancer. Representative drugs include osimertinib, lazertinib, and the like, which target the T790M mutation and show relatively low toxicity, and thus are clinically used to treat non-small cell lung cancer (J Thorac Dis. 2018 Jul 2018) ;10 (7) :3909-3921). However, drug resistance of the 3rd-generation EGFR inhibitors has been inevitably reported, and C797S mutation, MET amplification, and the like have been reported as main resistance mechanisms (J Hematol Oncol. 2016, Jul 22, 9(1): 59; Nature Medicine 2015, 21: 560-562; Lung Cancer 2018, 118: 105-110; and ASCO 2017, abstract 2572, 9020). The C797S mutation and MET amplification have been reported to be found separately but sometimes at the same time.

Anaplastic lymphoma kinase (ALK) gene abnormality (EML4-ALK transfusion) is observed in some patients with non-small cell lung cancer, and various tyrosine kinase inhibitors (TKIs) and the like are being used clinically to treat these cancers. ALK-positive non-small cell lung cancer is caused by the fusion of ALK and EML4 genes, and as the normally latent ALK gene accelerates the growth rate of cells by the fusion of the two genes, the cells receiving this signal rapidly metastasize to cancer cells. As a representative therapeutic drug, crizotinib was approved as a multi-targeted anticancer drug by the U.S. FDA in 2011. This drug is being used to treat metastatic ALK-positive non-small cell lung cancer and the like through inhibition of the activity of MET, ALK, ROS1, and the like. Looking at the results of the clinical study of crizotinib, patients with adenocarcinoma tissue-type lung cancer mainly participated, and 46% of them were Asian. It showed very good efficacy in that the tumor response rate was about 65% and the progression-free survival period was 7.7 months (3 months in the chemotherapy group), and the most common adverse events reported were visual field abnormality, diarrhea, vomiting, edema, nausea , and the like (J Thorac Oncol 2012;7(7):1086-90). When crizotinib is used, resistance inevitably occurs, and mainly secondary mutations in the ALK kinase domain (about 30%), ALK fusion gene mutation amplification, activation of bypass signaling pathway and the like have been reported. Although there are a wide variety of mutations, there are secondary mutations including L1196M and G1269A, and L1196M, which is located at the most frequent gate-keeper residue to induce hindrance of the binding of crizotinib to ALK (J Clin Oncol 2013;31(8):1105-11).

It is reported that all non-small cell lung cancers caused by ALK mutation or EGFR mutation (or both) have a secondary mutation that inhibits the kinase-drug binding force as the main resistance mechanism, and these mutations affect intracellular downstream signaling (Eur Med Chem. 2017 Aug 18;136:497-510). Despite the continuous development of various ALK and EGFR inhibitors, the development of inhibitors that inhibit the two kinases together is progressing very slowly. Thus, there is a need for the development of a drug that effectively inhibits the growth of ALK-mutated or EGFR-mutated cancer cells, which have the major drug resistance mechanisms described above.

In addition, the non-small cell lung cancer is caused by the expression, rearrangement and the like of various oncogenes, and examples include KRAS, ROS1, RET, and the like (Lancet Oncol 2011; 12 (2) : 175-80) .

### [Prior Art References]

### [Patent Documents]

PCT International Publication No. WO 2009/143389 A1

### [Disclosure]

### [Technical Problem]

The present inventors sought to develop a novel compound that effectively inhibits ALK-mutated and EGFR-mutated cancers. As a result, it was confirmed that a novel deuterium-substituted pyrimidine derivative exhibits excellent effects in the treatment of lung cancer.

Therefore, it is an object of the present invention to provide a novel deuterium-substituted pyrimidine derivative having an excellent effect in the treatment of lung cancer and a pharmaceutical composition comprising the same.

It is another object of the present invention to provide a novel deuterium-substituted pyrimidine derivative having an excellent effect in the treatment of lung cancer expressing ALK mutation or EGFR mutation among lung cancers and a pharmaceutical composition comprising the same.

### [Technical Solution]

In order to achieve the above objects, the present invention provides a compound represented by following Formula 1: 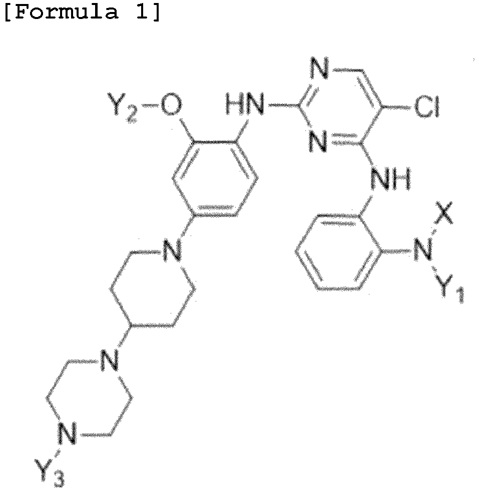 wherein,
X is a C1-C4 alkylsulfonyl group unsubstituted or substituted with deuterium or a C1-C4 dialkylphosphoryl group unsubstituted or substituted with deuterium; and
Y₁, Y₂, and Y₃ are each independently a C1-C4 alkyl group unsubstituted or substituted with deuterium,
wherein the compound of Formula 1 contains one or more deuteriums,
or a pharmaceutically acceptable salt thereof.

In addition, the present invention
provides a pharmaceutical composition for treating lung cancer, comprising the compound represented by Formula 1 above or a pharmaceutically acceptable salt thereof as an active ingredient, and a pharmaceutically acceptable carrier.

In addition, the present invention
provides the compound represented by Formula 1 above or a pharmaceutically acceptable salt thereof, which is used for the treatment of lung cancer.

In addition, the present invention
provides a method of treating an animal suffering from lung cancer, comprising administrating an effective amount of the compound represented by Formula 1 above or a pharmaceutically acceptable salt thereof to the animal.

### [Advantageous Effects]

The novel deuterium-substituted pyrimidine derivative of the present invention and the pharmaceutical composition comprising the same provide an excellent effect in the treatment of lung cancer.

In addition, the deuterium-substituted pyrimidine derivative and the pharmaceutical composition comprising the same effectively inhibit the growth of ALK-mutated or EGFR-mutated cancer cells in particular.

### [Description of Drawings]

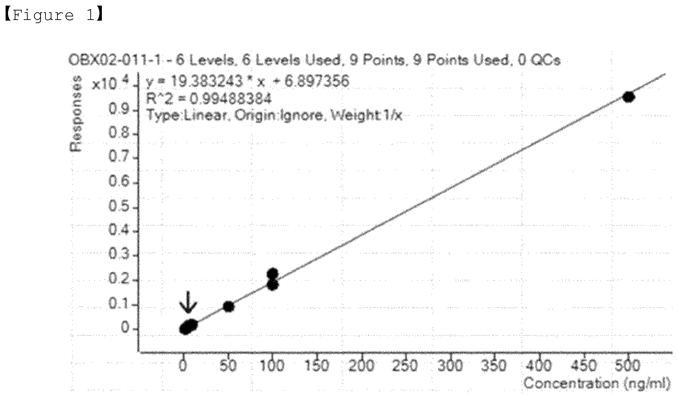

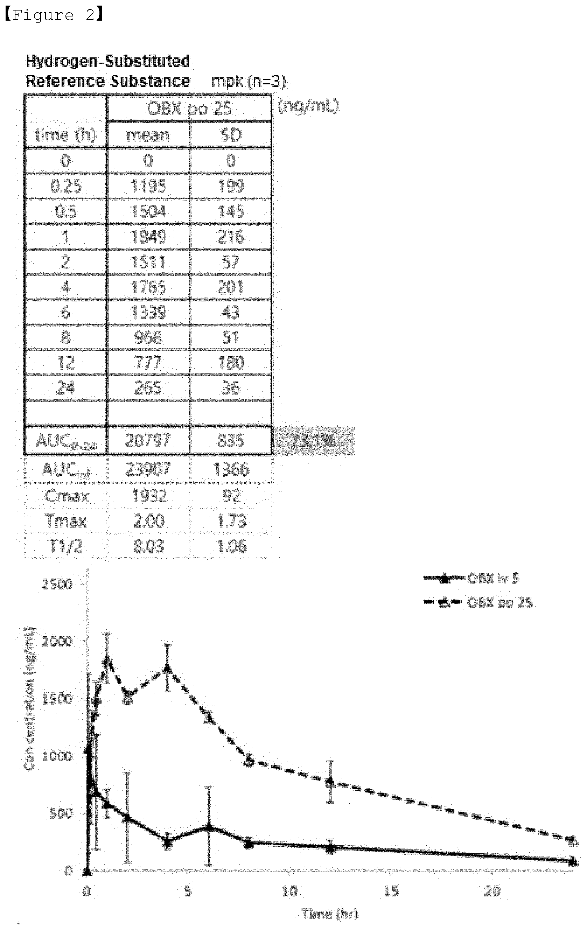

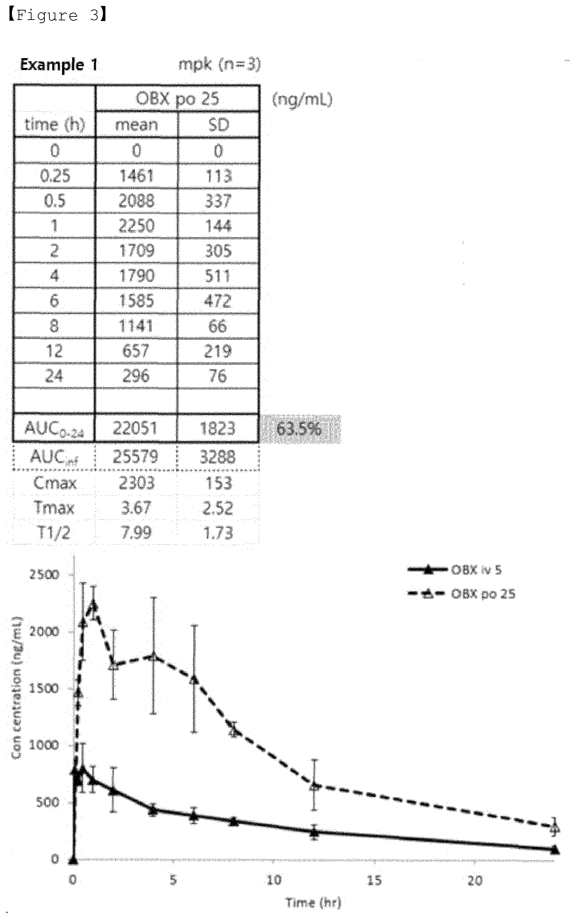

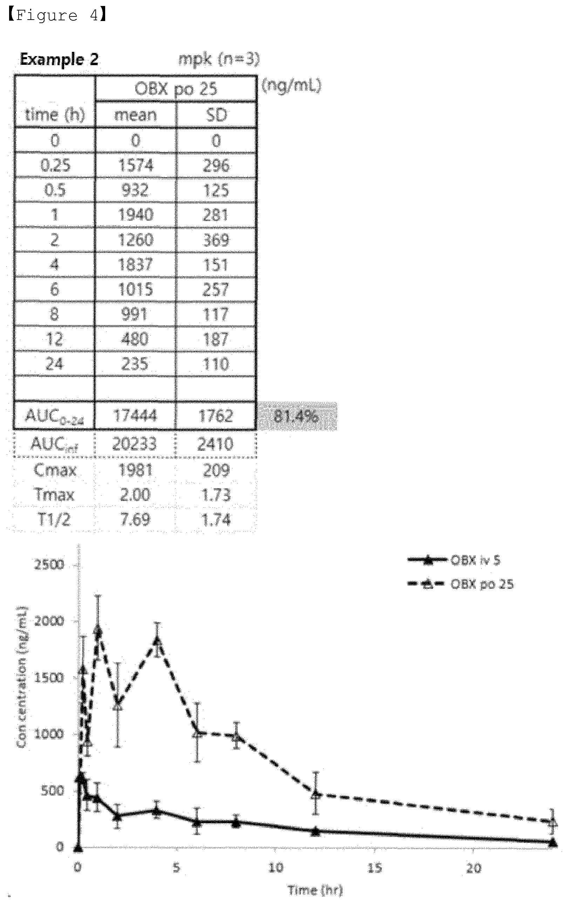

Figures 1 to 4 show the results of measuring pharmacokinetic elements in rats for the compounds obtained from Examples 1 and 2 of the present invention.

### [Best Mode]

Hereinafter, the present invention will be described in more detail with reference to embodiments. However, the present invention is not limited by the embodiments that have been represented by way of example, and the present invention is defined only by the scope of the appended claims. In addition, even if it is a constitution essential for practicing the present invention, a detailed description of the constitution that may be easily practiced by those skilled in the art will be omitted.

Unless otherwise stated below, the term "compound of the present invention" or "compound of Formula 1" is used as a concept including both the compound itself and a pharmaceutically acceptable salt thereof.

As used herein, the term "alkyl group" refers to straight and branched hydrocarbon groups having the specified number of carbon atoms. The alkyl group may be, for example, methyl, ethyl, n-propyl, i-propyl, n-butyl, s-butyl, i-butyl, t-butyl, and the like.

As used herein, the term "alkylsulfonyl" refers to alkylS(O₂)-. In this case, the alkyl is as defined above.

The present invention relates to a compound represented by following Formula 1: 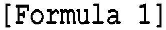 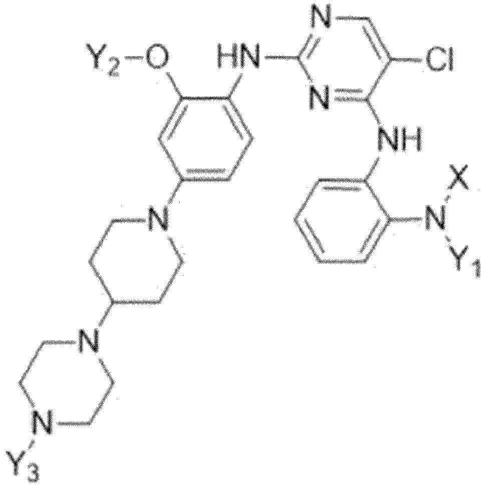 wherein,
X is a C1-C4 alkylsulfonyl group unsubstituted or substituted with deuterium or a C1-C4 dialkylphosphoryl group unsubstituted or substituted with deuterium; and
Y₁, Y₂, and Y₃ are each independently a C1-C4 alkyl group unsubstituted or substituted with deuterium,
wherein the compound of Formula 1 contains one or more deuteriums,
or a pharmaceutically acceptable salt thereof.

In the compound of Formula 1, any one or more of Y₁, Y₂, and Y₃ may be a C1-C4 alkyl group substituted with deuterium.

In the compound of Formula 1, the alkyl group in the C1-C4 alkyl group unsubstituted or substituted with the deuterium may be preferably a methyl group.

In the compound of Formula 1, X may be a C1-C4 alkylsulfonyl group unsubstituted or substituted with deuterium.

The compound of Formula 1 above is preferably any one of the following compounds:
N-(2-((5-chloro-2-((2-methoxy-4-(4-(4-(methyl-d3)piperazin-1-yl)piperidin-1-yl)phenyl)amino)pyrimidin-4-yl)amino)phenyl)-N-methylmethanesulfonamide (Compound 1);
N-(2-((5-chloro-2-((2-(methoxy-4-(4-(4-methylpiperazin-1-yl)piperidin-1-yl)phenyl)amino)pyrimidin-4-yl)amino)phenyl)-N-(methyl-d3)methanesulfonamide (Compound 2);
N-(2-((5-chloro-2-((2-(methoxy-d3)-4-(4-(4-methylpiperazin-1-yl)piperidin-1-yl)phenyl)amino)pyrimidin-4-yl) amino)phenyl)-N-methylmethanesulfonamide (Compound 3);
N-(2-((5-chloro-2-((2-methoxy-4-(4-(4-(methyl-d3)piperazin-1-yl)piperidin-1-yl)phenyl)amino)pyrimidin-4-yl)amino)phenyl)-N-(methyl-d3)methanesulfonamide (Compound 4);
N-(2-((5-chloro-2-((2-(methoxy-d3)-4-(4-(4-methylpiperazin-1-yl)piperidin-1-yl)phenyl)amino)pyrimidin-4-yl)amino)phenyl)-N-(methyl-d3)methanesulfonamide (Compound 5);
N-(2-((5-chloro-2-((2-methoxy-d3)-4-(4-(4-(methyl-d3)piperazin-1-yl)piperidin-1-yl)phenyl)amino)pyrimidin-4-yl)amino)phenyl)-N-methylmethanesulfonamide (Compound 6); and
N-(2-((5-chloro-2-((2-(methoxy-d3)-4-(4-(4-(methyl-d3)piperazin-1-yl)piperidin-1-yl)phenyl)amino)pyrimidin-4-yl)amino)phenyl)-N-(methyl-d3)methanesulfonamide (Compound 7).

The deuterium included in Formula 1 of the present invention is one of the isotopes of hydrogen and contains one proton and one neutron in a hydrogen atom, and thus has an atomic nucleus having twice the mass of general hydrogen. Deuterium, conventionally denoted by D, is an element that is stable, does not decay, and exists in very small amounts in nature. Deuterium is mainly produced by electrolyzing and concentrating water and is obtained by using the difference in the reaction rate in which light hard water reacts first with an electrode to decompose, and then heavy water reacts with the electrode at a relatively slow rate to electrolyze. In the case of a heavy element, it is not affected by the mass due to the number of neutrons, and thus, the difference in chemical properties between isotopes is not large. However, in the case of hydrogen with a small mass, a change in mass due to a change in the number of neutrons has a very sensitive effect on chemical and physical properties such as reactivity and diffusion rate. This is called the isotope effect, and it is known that it appears outstandingly in hydrogen and deuterium.

The present invention is characterized in that the anticancer activity of the compound of Formula 1 and a pharmaceutically acceptable salt thereof is improved by utilizing such properties of deuterium.

The salt of the compound represented by Formula 1 according to the present invention may be in the form of a salt derived from an inorganic acid or an organic acid, and in this case, preferred salts include hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, benzoic acid, hydroxybenzoic acid, phenylacetic acid, cinnamic acid, salicylic acid, methanesulfonic acid, ethanesulfonic acid, benzenesulfonic acid, toluenesulfonic acid, or the like.

In addition, the present invention relates to a pharmaceutical composition for treating lung cancer, comprising the compound represented by Formula 1 or a pharmaceutically acceptable salt thereof as an active ingredient, and a pharmaceutically acceptable carrier.

In particular, the pharmaceutical composition may be effectively used for the treatment of ALK-mutated and epidermal growth factor receptor (EGFR)-mutated lung cancers.

The pharmaceutical composition of the present invention may be used for both non-small cell lung cancer and small cell lung cancer.

The compound represented by Formula 1 according to the present invention may be used in the form of a pharmaceutically acceptable salt derived from an inorganic acid or an organic acid, and in this case, preferred salts include a salt derived from hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, benzoic acid, hydroxybenzoic acid, phenylacetic acid, cinnamic acid, salicylic acid, methanesulfonic acid, ethanesulfonic acid, benzenesulfonic acid, toluenesulfonic acid, or the like.

The pharmaceutical composition of the present invention may be formulated according to conventional methods, and may be prepared in various oral dosage forms such as tablets, pills, powders, capsules, syrups, emulsions, microemulsions, and the like, or in parenteral dosage forms such as intravenous infusion, subcutaneous infusion, intramuscular infusion, intraperitoneal infusion, transdermal infusion, and direct infusion into tissue.

When the pharmaceutical composition of the present invention is prepared in the form of an oral formulation, ingredients known in the art may be used without limitation as a pharmaceutically acceptable carrier, so long as they do not interfere with the active expression of the active ingredient.

The carrier may include, for example, excipients, diluents, disintegrants, binders, glidants, surfactants, emulsifiers, suspending agents, and the like, but is not limited thereto.

When the pharmaceutical composition of the present invention is prepared in the form of an injection, ingredients known in the art may be used without limitation as a pharmaceutically acceptable carrier, so long as they do not interfere with the active expression of the active ingredient.

Specifically, the carrier may include, for example, water, saline, aqueous glucose solution, aqueous pseudo-sugar solution, alcohol, glycol, ether (e.g., polyethylene glycol 400), oil, fatty acid, fatty acid ester, glyceride, surfactant, suspending agent, emulsifier, and the like, but is not limited thereto.

The dosage of the pharmaceutical composition of the present invention is preferably determined in consideration of the patient's age, sex, and condition, the degree of absorption of the active ingredient in the body, the inactivation rate, and the drug to be used in combination, and may be from 0.0001 mg/kg (body weight) to 100 mg/kg (body weight) per time based on the compound represented by Formula 1. It is appropriate that the number of administrations is about 1 to 3 times a day.

In addition, the present invention
relates to the compound represented by Formula 1 or a pharmaceutically acceptable salt thereof, which is used for the treatment of lung cancer.

In addition, the present invention relates to a method of treating an animal suffering from lung cancer, comprising administrating an effective amount of the compound represented by Formula 1 above or a pharmaceutically acceptable salt thereof to the animal.

The animal may be a human, and the lung cancer may be a lung cancer having ALK-mutated or EGFR-mutated cancer cell.

### [Mode for Carrying out the Invention]

Hereinafter, the present invention will be described in more detail through examples. It will be apparent to those skilled in the art that these examples are only for illustrating the present invention in more detail and the scope of the present invention is not limited to these examples in accordance with the gist of the present invention.

The compound represented by Formula 1 of the present invention may be prepared by the method illustrated in the following scheme, but is not limited thereto.

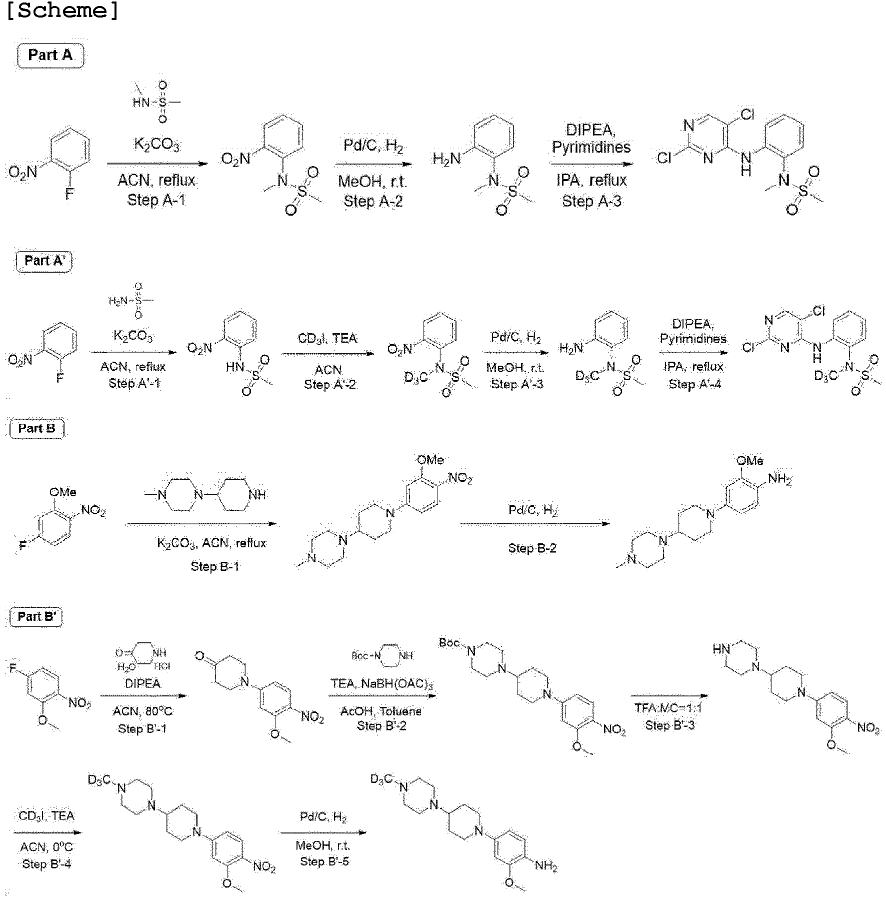

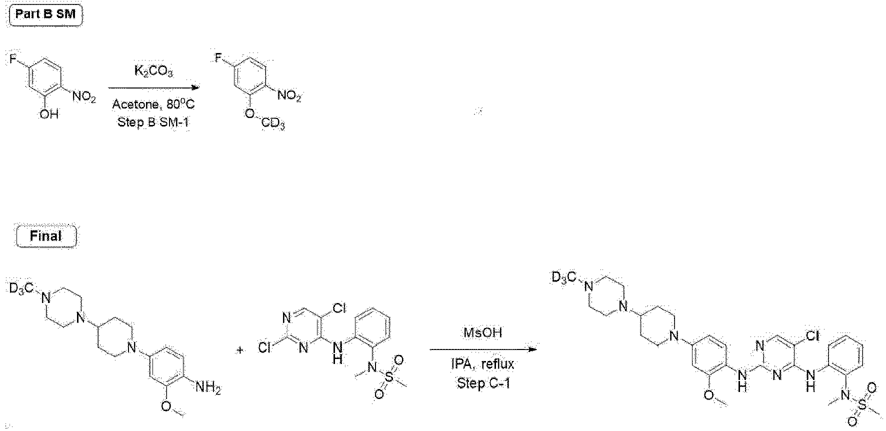

### Step A-1: Synthesis of N-methyl-N-(2-nitrophenyl)methansulfonamide

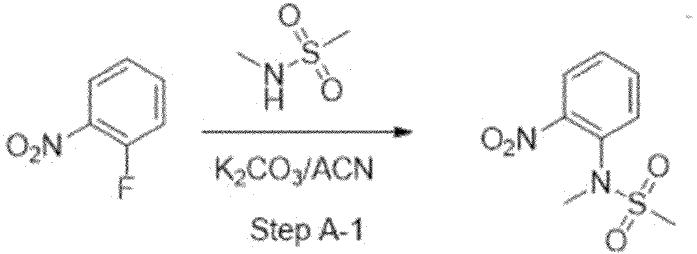

1-Fluoro-2-nitrobenzene (1.0 eq) is dissolved in acetonitrile, and potassium carbonate (2.0 eq) and N-methylmethanesulfonamide (1.4 eq) are added thereto at room temperature. Thereafter, it is stirred at 80°C overnight. After completion of the reaction, the temperature is lowered to room temperature and filtration is performed. The filtrate is evaporated under reduced pressure to obtain the compound, which is used in the next reaction without any separation process.

### Step A-2: Synthesis of N-(2-aminophenyl)-N-methylmethanesulfonamide

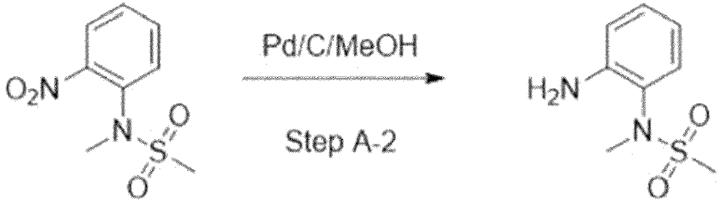

*N*-methyl-*N*-(2-nitrophenyl)methanesulfonamide (1.0 eq) is dissolved in a mixed solution of methanol and ethyl acetate (1:1), and 10% palladium/charcoal (0.2 eq) is added thereto. It is stirred for 2 hours under hydrogen. After completion of the reaction, it is filtered through celite. The filtrate is evaporated under reduced pressure, and solidified using ethyl ether and pentane. Thereafter, it is filtrated to obtain the target compound, which is used in the next reaction without any separation process.

### Step A-3: Synthesis of N-(2-((2,5-dichloropyrimidin-4-yl)amino)phenyl)-N-methylmethanesulfonamide

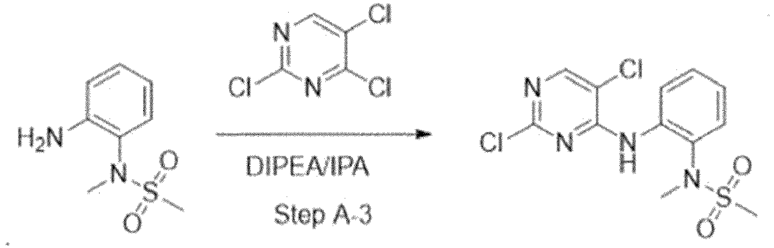

*N*-(2-aminophenyl)-*N*-methylmethanesulfonamide (1.0 eq) is dissolved in isopropyl alcohol, and 2,4,5-trichloropyrimidine (1.1 eq) and N,N-diisopropylethylamine (2.5 eq) are added thereto at room temperature. It is stirred at 80°C overnight. After completion of the reaction, it is evaporated under reduced pressure and extracted using water and dichloromethane. The organic layer is washed using 2N hydrochloric acid. The organic layer is evaporated under reduced pressure to obtain the target compound, which is used in the next reaction without any separation process.

### Step A'-1: Synthesis of N-(2-nitrophenyl)methanesulfonamide

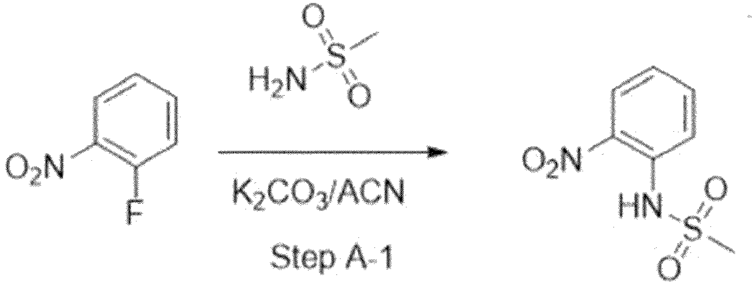

1-Fluoro-2-nitrobenzene (1.0 eq) is dissolved in acetonitrile, and potassium carbonate (2.0 eq) and methanesulfonamide (1.4 eq) are added thereto at room temperature. Thereafter, it is stirred at 80°C overnight. After completion of the reaction, the temperature is lowered to room temperature and filtration is performed. The filtrate is evaporated under reduced pressure to obtain the target compound, which is used in the next reaction without any separation process.

### Step A'-2: Synthesis of N-(methyl-d3)-N-(2-nitrophenyl)methanesulfonamide

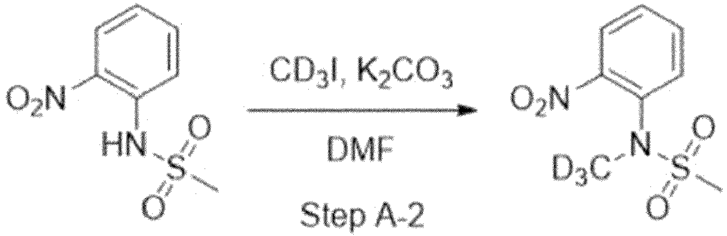

N-(2-nitrophenyl)methanesulfonamide (1.0 eq) is dissolved in N,N-dimethylformamide, and potassium carbonate (1.2 eq) and iodomethane-d3 (1.1 eq) are added thereto at room temperature. After it is stirred at 70°C for 2 hours, the reaction is completed, and the temperature is lowered to room temperature and water is added thereto. The resulting solid is filtered, washed with water sufficiently, and used in the next reaction without any separation process.

### Step A'-3: Synthesis of N-(2-aminophenyl)-N-(methyl-d3)methanesulfonamide

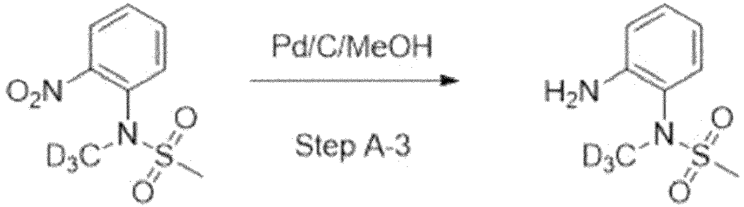

*N*-methyl-*N*-(2-nitrophenyl)methanesulfonamide (1.0 eq) is dissolved in methanol and ethyl acetate (1:1), and 10% palladium/charcoal (0.2 eq) is added thereto. It is stirred for 2 hours under hydrogen. After completion of the reaction, it is filtered through celite. The filtrate is evaporated under reduced pressure. After it is solidified using ethyl ether and pentane, it is filtrated to obtain the target compound, which is used in the next reaction without any separation process.

### Step A'-4: Synthesis of N-(2-((2,5-dichloropyrimidin-4-yl)amino)phenyl)-N-(methyl-d3)methanesulfonamide

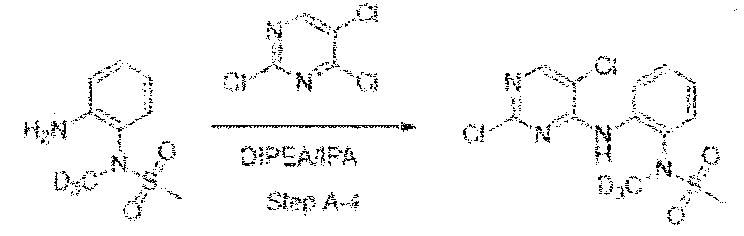

*N*-(2-aminophenyl)-*N*-methylmethanesulfonamide (1.0 eq) is dissolved in isopropyl alcohol, and 2,4,5-trichloropyrimidine (1.1 eq) and N,N-diisopropylethylamine (2.5 eq) are added thereto at room temperature. It is stirred at 80°C overnight. After completion of the reaction, it is evaporated under reduced pressure and extracted using water and dichloromethane. The organic layer is washed with 2 N hydrochloric acid, and the organic layer is evaporated under reduced pressure to obtain the target compound. It is used in the next reaction without any separation process.

### Step B-1: Synthesis of 1-(1-(3-methoxy-4-nitrophenyl)piperidin-4-yl)-4-methylpiperazine

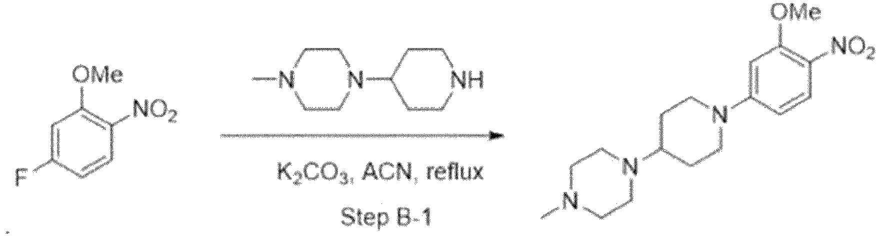

4-Fluoro-2-methoxy-1-nitrobenzene (1.0 eq) is dissolved in acetonitrile, and potassium carbonate (2.5 eq) and piperazine intermediate (1.1 eq) are added thereto at room temperature. It is stirred overnight under reflux. After completion of the reaction, the temperature is lowered to room temperature and filtration is performed. The filtrate is evaporated under reduced pressure to obtain the target compound, which is used in the next reaction without any separation process.

### Step B-2: Synthesis of 2-methoxy-4-(4-(4-methylpiperazin-1-yl)piperidin-1-yl)aniline

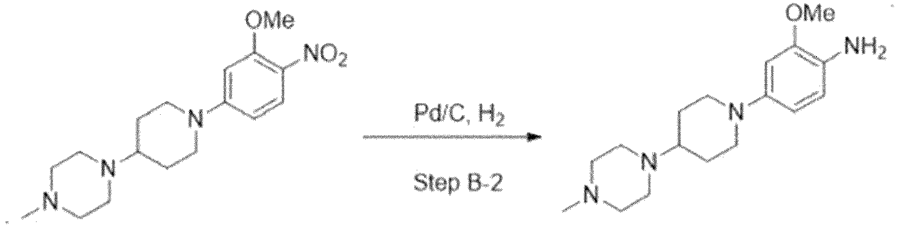

1-(1-(3-Methoxy-4-nitrophenyl)piperidin-4-yl)-4-methylpiperazine (1.0 eq) is dissolved in a mixed solvent of methyl alcohol and dichloromethane (1:1), and 10% palladium/charcoal (0.2 eq) is added thereto. It is stirred for 2 hours under hydrogen. After completion of the reaction, it is filtered through celite, and the filtrate is evaporated under reduced pressure. It is solidified using hexane, and the resulting solid is used in the next reaction without any separation process.

### Step B'-1: Synthesis of 1-(3-methoxy-4-nitrophenyl)piperidin-4-one

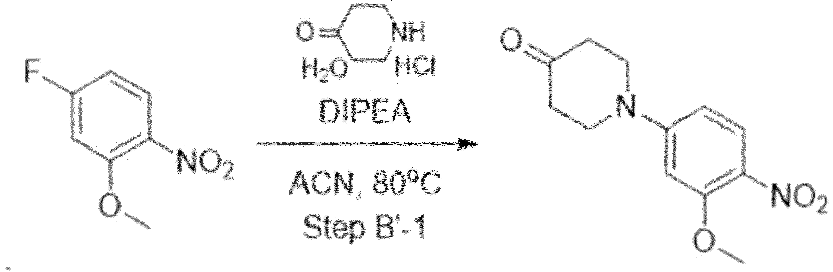

4-Fluoro-2-methoxy-1-nitrobenzene (1.0 eq) is dissolved in acetonitrile. N,N-diisopropylethylamine (3.0 eq) and 4-piperidone monohydrate hydrochloride (1.2 eq) are added thereto at room temperature. It is stirred at 80°C overnight. After completion of the reaction, the solvent is evaporated under reduced pressure, and it is extracted with water and dichloromethane. The organic layer is collected and evaporated under reduced pressure to obtain the target compound. It is used in the next reaction without any particular separation process.

### Step B'-2: Synthesis of tert-butyl 4-(1-(3-methoxy-4-nitrophenyl)piperidin-4-yl)piperazine-1-carboxylate

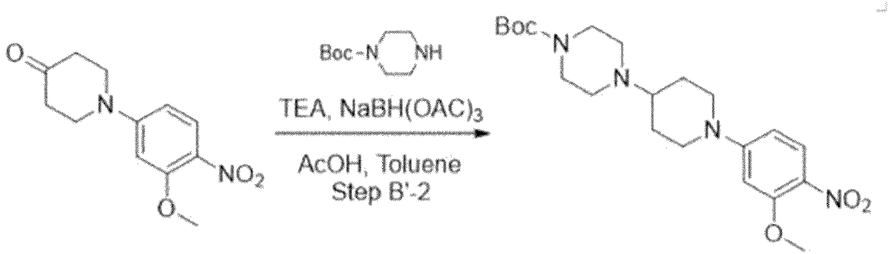

1-(3-Methoxy-4-nitrophenyl)piperidin-4-one (1.0 eq) is dissolved in toluene, and tert-butyl piperazine-1-carboxylate (1.97 eq), triethylamine (2.58 eq), and acetic acid (1.53 eq) are added thereto at room temperature. After it is stirred at room temperature for 30 minutes, sodium triacetoxyborohydride (0.83 eq) is added thereto. This process is repeated two more times. After completion of the addition, it is stirred at room temperature overnight. After completion of the reaction, it is extracted with water and ethyl acetate, and the organic layer is collected. The organic layer is evaporated under reduced pressure to obtain the title compound. It is used in the next reaction without any separation process.

### Step B'-3: Synthesis of 1-(1-(3-methoxy-4-nitrophenyl)piperidin-4-yl)piperazine

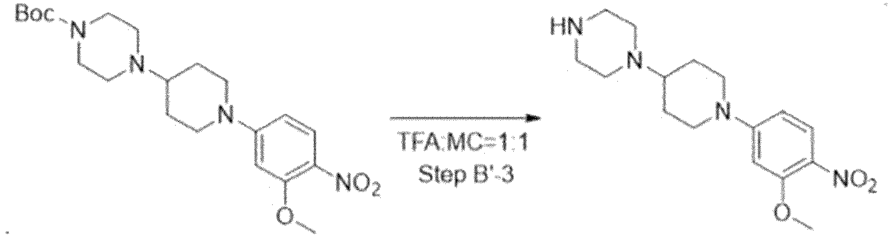

Tert-butyl 4-(1-(3-methoxy-4-nitrophenyl)piperidin-4-yl)piperazine-1-carboxylate (1.0 eq) is dissolved in a mixed solvent of trifluoroacetic acid and dichloromethane (1:1) and stirred at room temperature. After completion of the reaction, it is evaporated under reduced pressure. It is extracted with 2 N potassium hydroxide solution and dichloromethane, and the organic layer is collected. The organic layer is evaporated under reduced pressure to obtain the title compound. It is used in the next reaction without any separation process.

### Step B'-4: Synthesis of 1-(1-(3-methoxy-4-nitrophenyl)piperidin-4-yl)-4-(methyl-d3)piperazine

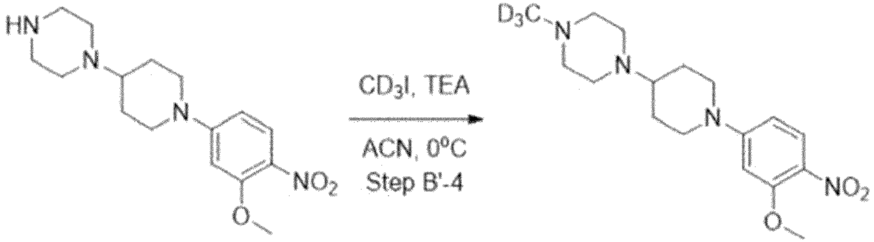

1-(1-(3-Methoxy-4-nitrophenyl)piperidin-4-yl)piperazine (1.0 eq) is dissolved in acetonitrile, and triethylamine (1.2 eq) and iodomethane-d3 (1.1 eq) are added thereto at 0°C. It is stirred at the same temperature. After completion of the reaction, it is extracted with water and ethyl acetate, and the organic layer is collected. The organic layer is evaporated under reduced pressure, and the target compound is obtained using column chromatography (10% methyl alcohol/dichloromethane).

### Step B'-5: Synthesis of 2-methoxy-4-(4-(4-(methyl-d3)piperazin-1-yl)piperidin-1-yl)aniline

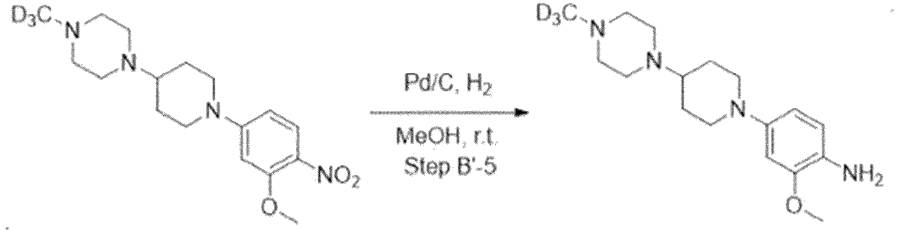

1-(1-(3-Methoxy-4-nitrophenyl)piperidin-4-yl)-4-(methyl-d3)piperazine (1.0 eq) is dissolved in a mixed solvent of methyl alcohol and ethyl acetate (1:1), and 10% palladium/charcoal (0.2 eq) is added thereto. It is stirred for 2 hours under hydrogen. After completion of the reaction, it is filtered through celite. The filtrate is evaporated under reduced pressure. It is solidified using hexane, and the resulting solid is used in the next reaction without any separation process.

### Step B SM-1: 4-Fluoro-2-(methoxy-d3)-1-nitrobenzene

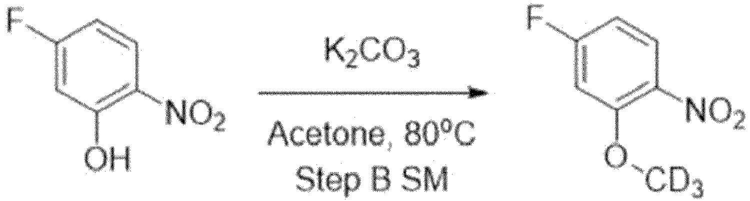

1-(1-(3-Methoxy-4-nitrophenyl)piperidin-4-yl)piperazine (1.0 eq) is dissolved in acetonitrile, and potassium carbonate (2.0 eq) and iodomethane-d3 (1.3 eq) are added thereto at room temperature. It is stirred at 60°C for 2 hours. After completion of the reaction, it is evaporated under reduced pressure and extracted with water and ethyl acetate, and the organic layer is collected. The organic layer is evaporated under reduced pressure, and the target compound is obtained using column chromatography (25% ethyl acetate/n-hexane).

### Step C-1: Synthesis of final compound

Pyrimidine derivative (1.0 eq) is dissolved in isopropyl alcohol, and aniline derivative (1.0 eq) and methanesulfonyl acid (1.3 eq) are added thereto at room temperature. It is stirred at 80°C overnight. After completion of the reaction, it is evaporated under reduced pressure to remove the solvent, and extracted with water and a 10% mixed solution of methanol/dichloromethane. The organic layer is evaporated under reduced pressure, and the target compound is obtained using column chromatography (10% methyl alcohol/dichloromethane) .

### Example 1: N-(2-((5-Chloro-2-((2-methoxy-4-(4-(4-(methyl-d3)piperazin-1-yl)piperidin-1-yl)phenyl)amino)pyrimidin-4-yl)amino)phenyl)-N-methylmethanesulfonamide (Compound 1)

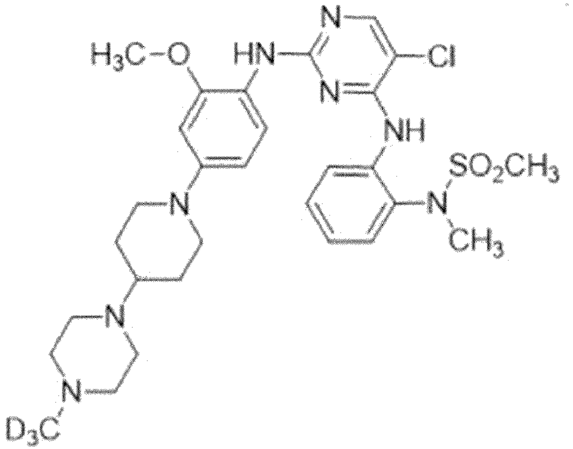

**Step A, Step B',** and **Step C** were used to synthesize the compound.

Yield: 22.5%, White solid,
1H NMR(400 MHz, DMSO-d6) δ 8.23(m, 2H), 8.07 - 8.06(m, 2H), 7.54(dd, J = 7.9, 1.6 Hz, 1H), 7.32(d, J = 8.6 Hz, 1H), 7.21(t, J = 7.8 Hz, 1H), 7.12(td, J = 7.6, 1.5 Hz, 1H), 6.57(d, J = 2.5 Hz, 1H), 6.41(dd, J = 8.7, 2.6 Hz, 1H), 3.71- 3.66(m, 5H), 3.14(s, 3H), 3.06(s, 3H), 2.62(t, J = 11.7 Hz, 3H), 2.52 - 2.43(m, 4H), 2.29 - 2.23(m, 5H), 1.85 - 1.79(m, 2H), 1.54 - 1.41(m, 2H). MS: ESI m/z 618.20 [M+H]+

### Example 2: N-(2-((5-Chloro-2-((2-(methoxy-4-(4-(4-methylpiperazin-1-yl)piperidin-1-yl)phenyl)amino)pyrimidin-4-yl)amino)phenyl)-N-(methyl-d3)methanesulfonamide (Compound 2)

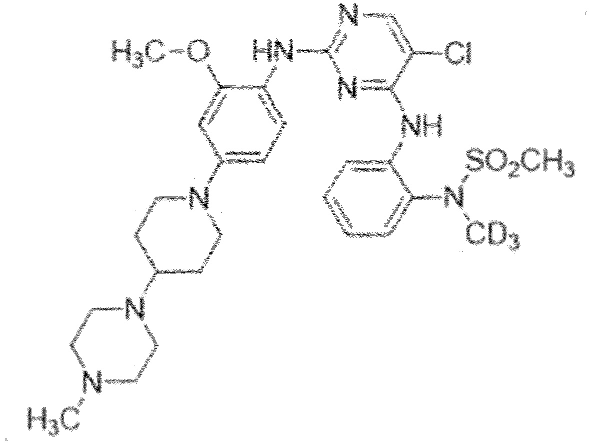

**Step A', Step B,** and **Step C** were used to synthesize the compound.

Yield: 25.5%, White solid,
1H NMR(400 MHz, DMSO-d6) δ 8.24 - 8.22(m, 2H), 8.06(s, 2H), 7.54(dd, J = 7.9, 1.6 Hz, 1H), 7.32(d, J = 8.6 Hz, 1H), 7.21(t, J = 7.7 Hz, 1H), 7.12(td, J = 7.6, 1.5 Hz, 1H), 6.58(d, J = 2.5 Hz, 1H), 6.41(dd, J = 8.8, 2.6 Hz, 1H), 3.71 - 3.66(m, 5H), 3.06(s, 3H), 2.65 - 2.60(m, 2H), 2.53 - 2.43(m, 4H), 2.29 - 2.24(m, 5H), 2.10(s, 3H), 1.86 - 1.79(m, 2H), 1.53 - 1.43(m, 2H). MS: ESI m/z 618.20 [M+H]+

### Example 3: N-(2-((5-Chloro-2-((2-(methoxy-d3)-4-(4-(4-methylpiperazin-1-yl)piperidin-1-yl)phenyl)amino)pyrimidin-4-yl)amino)phenyl)-N-methylmethanesulfonamide (Compound 3)

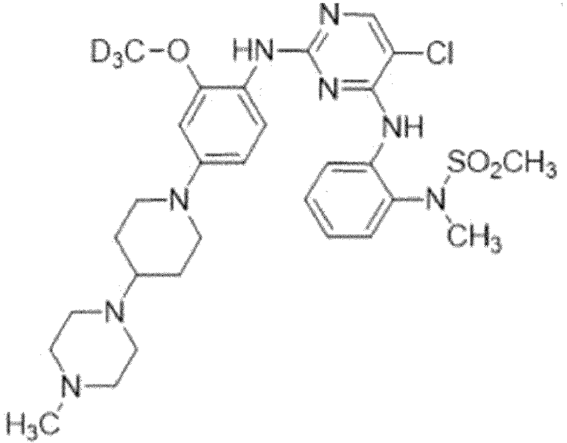

**Step A', Step B SM, Step B,** and **Step C** were used to synthesize the compound.

Yield: 40.3%, White solid,
1H NMR(400 MHz, DMSO-d6) δ 8.23(m, 2H), 8.07 - 8.06(m, 2H), 7.54(dd, J = 7.9, 1.6 Hz, 1H), 7.32(d, J = 8.6 Hz, 1H), 7.21(t, J = 7.8 Hz, 1H), 7.12(td, J = 7.6, 1.5 Hz, 1H), 6.57(d, J = 2.5 Hz, 1H), 6.41(dd, J = 8.7, 2.6 Hz, 1H), 3.70- 3.66(m, 2H), 3.14(s, 3H), 3.06(s, 3H), 2.62(t, J = 11.7 Hz, 3H), 2.52 - 2.43(m, 4H), 2.29 - 2.23(m, 5H), 2.10(s, 3H), 1.85 - 1.79(m, 2H), 1.54 - 1.41(m, 2H). MS: ESI m/z 618.20 [M+H]+

### Example 4: N-(2-((5-Chloro-2-((2-methoxy-4-(4-(4-(methyl-d3)piperazin-1-yl)piperidin-1-yl)phenyl)amino)pyrimidin-4-yl)amino)phenyl)-N-(methyl-d3)methanesulfonamide

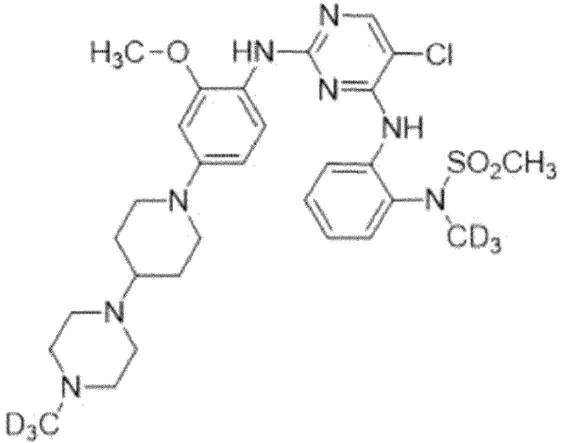

**Step A', Step B',** and **Step C** were used to synthesize the compound.

Yield: 20.2%, White solid, 1H NMR(400 MHz, DMSO-d6) δ 8.26 - 8.23(m, 2H), 8.08 - 8.06(m, 2H), 7.54(dd, J = 7.9, 1.6 Hz, 1H), 7.32 (d, J = 8.6 Hz, 1H), 7.21(t, J = 7.7 Hz, 1H), 7.12(td, J = 7.6, 1.5 Hz, 1H), 6.58(d, J = 2.5 Hz, 1H), 6.41(dd, J = 8.8, 2. 6 Hz, 1H), 3.71 - 3.66(m, 5H), 3.06(s, 3H), 2.66 - 2.59(m, 2H), 2.53 - 2.43(m, 4H), 2.37 - 2.25(m, 5H), 1.87 - 1.80(m, 2H), 1.52 - 1.44(m, 2H). MS: ESI m/z 621.20 [M+H]+

### Example 5: N-(2-((5-Chloro-2-((2-(methoxy-d3)-4-(4-(4-methylpiperazin-1-yl)piperidin-1-yl)phenyl)amino)pyrimidin-4-yl)amino)phenyl)-N-(methyl-d3)methanesulfonamide

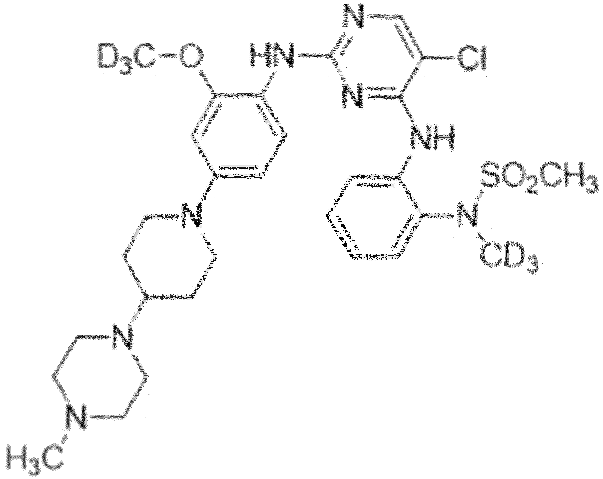

**Step A', Step B SM, Step B,** and **Step C** were used to synthesize the compound.

Yield: 38.9%, White solid,
1H NMR(400 MHz, DMSO-d6) δ 8.26 - 8.23(m, 2H), 8.08 - 8.06(m, 2H), 7.54(dd, J = 7.9, 1.6 Hz, 1H), 7.32 (d, J = 8.6 Hz, 1H), 7.21(t, J = 7.7 Hz, 1H), 7.12(td, J = 7.6, 1.5 Hz, 1H), 6.58(d, J = 2.5 Hz, 1H), 6.41(dd, J = 8.8, 2.6 Hz, 1H), 3.70 - 3.66(m, 2H), 3.06(s, 3H), 2.66 - 2.59(m, 2H), 2.53 - 2.43(m, 4H), 2.37 - 2.25(m, 5H), 2.10(s, 3H), 1.87 - 1.80(m, 2H), 1.52 - 1.44(m, 2H). MS: ESI m/z 621.20 [M+H]+

### Example 6: N-(2-((5-Chloro-2-((2-methoxy-4-(4-(4-(methyl-d3)piperazin-1-yl)piperidin-1-yl)phenyl)amino)pyrimidin-4-yl)amino)phenyl)-N-(methyl-d3)methanesulfonamide

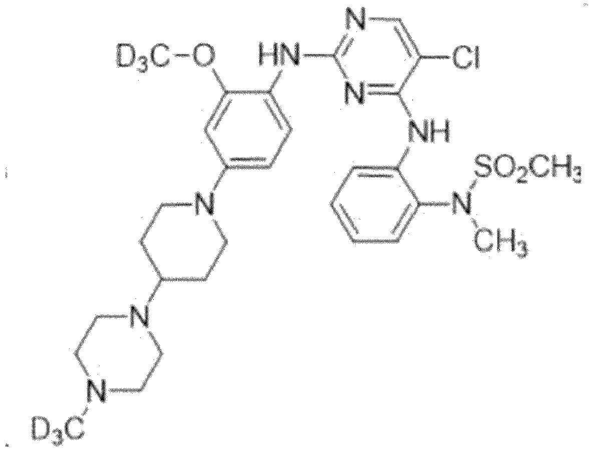

**Step A, Step B SM, Step B',** and **Step C** were used to synthesize the compound.

Yield: 41.3%, White solid,
1H NMR(400 MHz, DMSO-d6) δ 8.26 - 8.23(m, 2H), 8.08 - 8.06(m, 2H), 7.54 (dd, J = 7.9, 1.6 Hz, 1H), 7.32 (d, J = 8.6 Hz, 1H), 7.21(t, J = 7.7 Hz, 1H), 7.12(td, J = 7.6, 1.5 Hz, 1H), 6.58(d, J = 2.5 Hz, 1H), 6.41(dd, J = 8.8, 2.6 Hz, 1H), 3.70 - 3.66(m, 2H), 3.14(s, 3H), 3.06(s, 3H), 2.66 - 2.59(m, 2H), 2.53 - 2.43(m, 4H), 2.37 - 2.25(m, 5H), 1.87 - 1.80(m, 2H), 1.52 - 1.44(m, 2H). MS: ESI m/z 621.20 [M+H]+

### Example 7: N-(2-((5-Chloro-2-((2-(methoxy-d3)-4-(4-(4-(methyl-d3)piperazin-1-yl)piperidin-1-yl)phenyl)amino)pyrimidin-4-yl)amino)phenyl)-N-(methyl-d3)methanesulfonamide (Compound 7)

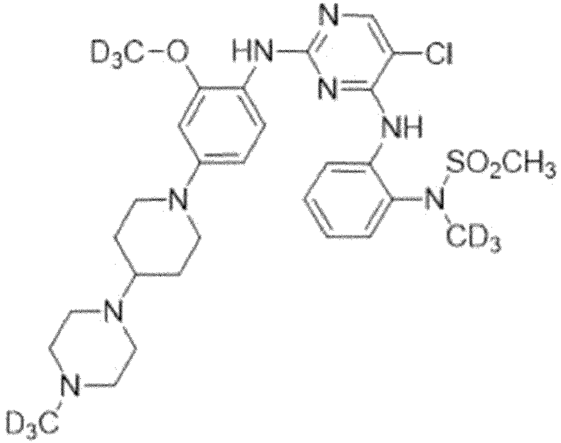

**Step A', Step B SM, Step B',** and **Step C** were used to synthesize the compound.

Yield: 38.1%, White solid,
1H NMR(400 MHz, DMSO-d6) δ 8.26 - 8.23(m, 2H), 8.08 - 8.06(m, 2H), 7. 54 (dd, J = 7. 9, 1.6 Hz, 1H), 7.32 (d, J = 8.6 Hz, 1H), 7.21(t, J = 7.7 Hz, 1H), 7.12(td, J = 7.6, 1.5 Hz, 1H), 6.58(d, J = 2.5 Hz, 1H), 6.41(dd, J = 8.8, 2.6 Hz, 1H), 3.70 - 3.66(m, 2H), 3.06(s, 3H), 2.66 - 2.59(m, 2H), 2.53 - 2.43(m, 4H), 2.37 - 2.25(m, 5H), 1.87 - 1.80(m, 2H), 1.52 - 1.44(m, 2H). MS: ESI m/z 624.30 [M+H]+

### Example 8: Synthesis of N-(2-((5-chloro-2-((2-methoxy-4-(4-(4-methylpiperazin-1-yl)piperidin-1-yl)phenyl)amino)pyrimidin-4-yl)amino)phenyl)-N-(methyl-d3)methanesulfonamide methanesulfonate

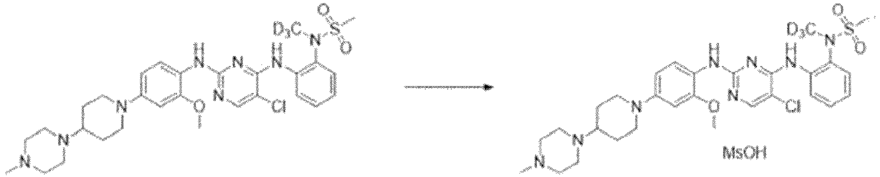

N-(2-((5-Chloro-2-((2-methoxy-4-(4-(4-methylpiperazin-1-yl)piperidin-1-yl)phenyl)amino)pyrimidin-4-yl)amino)phenyl)-N-(methyl-d3)methanesulfonamide (compound prepared in Example 2, 24.0 mg, 0.039 mmol) was dissolved in ethyl alcohol (1 mL), and methanesulfonic acid (3.8 mg, 0.039 mmol) was added thereto at room temperature. Thereafter, it was stirred until a solid was formed. When a solid was formed, heptane (1 mL) was added thereto and stirred for 2 hours. After completion of stirring, it was filtered and washed with heptane. The filtered solid was dried at 70°C to synthesize the target compound.

Yield: 97.4%; Light gray powder; 1H NMR (400 MHz, DMSO-d6) δ 8.35 (s, 1H), 8.20 (m, 2H), 8.08 (s, 1H), 7.56 (dd, J = 7.9, 1.5 Hz, 1H), 7.36 (s, 1H), 7.24 (m, 1H), 7.15 (t, J = 7.6 Hz, 1H), 6.63 (s, 1H), 6.47 (s, 1H), 3.83 - 3.65 (m, 5H), 3.10 - 2.89 (s, 7H), 2.84 - 2.63 (s, 6H), 2.31 - 2.27 (m, 1H), 2.27 (s, 3H), 1.91 (d, J = 32.1 Hz, 2H), 1.58 (s, 2H), 1.31 - 1.07 (m, 2H). MS: ESI m/z 714.05 [M+H]+

### Test Example 1: Measurement of inhibitory effect on cancer cell growth

The inhibitory activity of epithelial growth factor receptor (EGFR) kinase containing C797S was measured for the compounds obtained from Examples 1 and 2 above, the compound in which deuterium is substituted with hydrogen in the compound of Example 2 (hydrogen-substituted reference substance), and the control drug 3rd-generation osimertinib, and the results are shown in Table 1 below. The method of measuring kinase inhibitory activity was as follows:
1. Each kinase was incubated under 8 mM MOPS (pH 7.0), 0.2 mM EDTA, 250 µM KKKGQEEEYVFIE, 1 mM sodium orthovanadate, 5 mM sodium-6-glycerophosphate, 10 mM magnesium acetate, and [η-³³P]-ATP.
2. The compound to be evaluated (DMSO solution) and Mg/ATP were added thereto to proceed the reaction.
3. After about 40 minutes at room temperature, 10 µL of 0.5% phosphoric acid was added thereto to complete the reaction.
4. 0.5% reaction solution was divided into 10 µL and spotted on P30 filtermat.
5. It was washed 4 times with 0.425% phosphoric acid for about 4 minutes.
6. It was washed once with methanol, and then dried and analyzed by scintillation counting to measure IC₅₀ value.
7. Each compound was calculated as a GI₅₀ value, which is the concentration to inhibit 50% of cell growth, and the results are shown as A, B, C, and D in Table 1 below.

### <Evaluation Criteria>

A: GI₅₀ ≤ 500 nM, B: 500 nM < GI₅₀ ≤ 1, 000 nM, C: GI₅₀ > 1,000 nM

**[Table 1]**

| Example | BaF3(del19/T790M/C797S) |
|---|---|
| Compound of Example 1 | A |
| Compound of Example 2 | A |
| Compound of Example 2 in which deuterium is substituted with hydrogen (hydrogen-substituted reference substance) | A |
| Osimertinib | C |

As shown in Table 1 above, Compounds 1 and 2 prepared in Examples 1 and 2 of the present invention showed excellent activity against cancer cell lines expressing the C797S mutant epidermal growth factor receptor. On the other hand, osimertinib, which is the 3rd-generation anticancer agent, has poor activity. Thus, it may be confirmed from these experimental results that the compounds of the present invention are novel pyrimidine derivatives capable of treating C797S-mutated lung cancer caused by the resistance mechanism of the existing 3rd-generation drugs.

### Test Example 2: Pharmacokinetic evaluation

Pharmacokinetic tests were carried out in rats as follows for the compounds obtained from Examples 1 and 2 above and the compound of Example 2 in which deuterium was substituted with hydrogen (hydrogen-substituted reference substance).

After the test substances were prepared to 2.5 or 5 mg/mL using WFI (water for injection), the rats were administered as a single dose at a prescribed dose (10 mL/kg), and blood was collected at a predetermined time (0, 0.25, 0.5, 1, 2, 4, 6, 8, 10, 24 hours), and then plasma was separated. Analysis of the drug was carried out using HPLC (XBridge column C18, Waters, mobile phase of 0.1% formic acid:acetonitrile (30:70, %/%)) and MS/MS (ESI positive, MRM), and concentrations of 5, 50, 100, 500, 1,000, and 5,000 ng/ml were prepared and measured by mixing rat blank plasma and each commercial standard solution at a ratio of 9:1. In addition, QC sample preparations were prepared at concentrations of 100, 750, and 2,500 ng/ml by mixing rat blank plasma and QC standard solution in a 9:1 ratio. As for the pretreatment method, 100 ul of the plasma sample was transferred to a centrifuge tube, and 10 µl of the internal standard solution and 300 µl of methanol were added thereto, and then mixed for about 30 seconds. The tube was centrifuged for about 5 minutes at a rotation speed of 12,000 rpm (4°C) using a centrifuge, and the supernatant was taken, transferred to an LC vial and then injected into the device. Thereafter, the concentration of the drug in rat plasma was quantified by applying the previously verified assay. For pharmacokinetic parameters, WinNonlin 5.2 (Pharsight, USA) program was used, and AUC0-t, AUC0-∞, Cmax, Tmax, and t1/2 were calculated by noncompartment modeling (best fit). Pharmacokinetic parameter results were expressed as mean and standard deviation (SD), and statistically processed using the SPSS program (Statistical Package for the Social Sciences, 10.0K, USA).

The experimental results are shown in Figures 1 to 4. As shown in Figures 1 to 4 above, the compounds of Examples 1 and 2 exhibited an absolute absorption rate equivalent to or greater than the control drug, the compound of Example 2 in which deuterium is substituted with hydrogen (hydrogen-substituted reference substance). In particular, the compound of Example 2 exhibited an improved relative absorption rate of 111.4% compared to the hydrogen-substituted reference substance.

## Claims

1. A compound represented by following Formula 1: 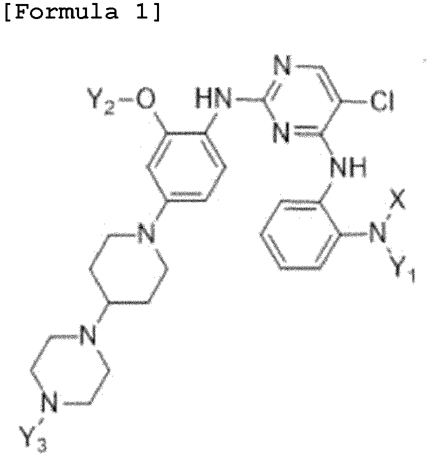 wherein,
X is a C1-C4 alkylsulfonyl group unsubstituted or substituted with deuterium or a C1-C4 dialkylphosphoryl group unsubstituted or substituted with deuterium; and
Y₁, Y₂, and Y₃ are each independently a C1-C4 alkyl group unsubstituted or substituted with deuterium,
wherein the compound of Formula 1 contains one or more deuteriums,
or a pharmaceutically acceptable salt thereof.

2. The compound represented by following Formula 1 or a pharmaceutically acceptable salt thereof according to claim 1, **characterized in that** any one or more of Y₁, Y₂, and Y₃ are a C1-C4 alkyl group substituted with deuterium.

3. The compound represented by following Formula 1 or a pharmaceutically acceptable salt thereof according to claim 1, **characterized in that** the alkyl group in the C1-C4 alkyl group unsubstituted or substituted with deuterium is a methyl group.

4. The compound represented by following Formula 1 or a pharmaceutically acceptable salt thereof according to claim 1, **characterized in that** X is a C1-C4 alkylsulfonyl group unsubstituted or substituted with deuterium.

5. The compound represented by following Formula 1 according to claim 1, **characterized in that** the compound of Formula 1 is any one of the following compounds:
N-(2-((5-chloro-2-((2-methoxy-4-(4-(4-(methyl-d3)piperazin-1-yl)piperidin-1-yl)phenyl) amino)pyrimidin-4-yl) amino)phenyl)-N-methylmethanesulfonamide (Compound 1);
N-(2-((5-chloro-2-((2-(methoxy-4-(4-(4-methylpiperazin-1-yl)piperidin-1-yl)phenyl) amino)pyrimidin-4-yl) amino)phenyl)-N-(methyl-d3)methanesulfonamide (Compound 2);
N-(2-((5-chloro-2-((2-(methoxy-d3)-4-(4-(4-methylpiperazin-1-yl)piperidin-1-yl)phenyl) amino)pyrimidin-4-yl) amino)phenyl)-N-methylmethanesulfonamide (Compound 3);
N-(2-((5-chloro-2-((2-methoxy-4-(4-(4-(methyl-d3)piperazin-1-yl)piperidin-1-yl)phenyl) amino)pyrimidin-4-yl) amino)phenyl)-N-(methyl-d3)methanesulfonamide (Compound 4);
N-(2-((5-chloro-2-((2-(methoxy-d3)-4-(4-(4-methylpiperazin-1-yl)piperidin-1-yl)phenyl) amino)pyrimidin-4-yl) amino)phenyl)-N-(methyl-d3)methanesulfonamide (Compound 5);
N-(2-((5-chloro-2-((2-methoxy-d3)-4-(4-(4-(methyl-d3)piperazin-1-yl)piperidin-1-yl)phenyl)amino)pyrimidin-4-yl)amino)phenyl)-N-methylmethanesulfonamide (Compound 6); and
N-(2-((5-chloro-2-((2-(methoxy-d3)-4-(4-(4-(methyl-d3)piperazin-1-yl)piperidin-1-yl)phenyl)amino)pyrimidin-4-yl)amino)phenyl)-N-(methyl-d3)methanesulfonamide (Compound 7), or a pharmaceutically acceptable salt thereof.

6. The compound represented by following Formula 1 or a pharmaceutically acceptable salt thereof according to claim 1, **characterized in that** the salt is a salt derived from one or more acids selected from the group consisting of hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, benzoic acid, hydroxybenzoic acid, phenylacetic acid, cinnamic acid, salicylic acid, methanesulfonic acid, ethanesulfonic acid, benzenesulfonic acid, and toluenesulfonic acid.

7. A pharmaceutical composition for treating lung cancer, comprising the compound of any one of claims 1 to 6 or a pharmaceutically acceptable salt thereof as an active ingredient, and a pharmaceutically acceptable carrier.

8. The pharmaceutical composition for treating lung cancer according to claim 7, **characterized in that** the pharmaceutically acceptable carrier is one or more selected from the group consisting of excipients, diluents, disintegrates, binders, lubricants, surfactants, emulsifiers, and suspending agents.

9. The pharmaceutical composition for treating lung cancer according to claim 7, **characterized in that** the pharmaceutically acceptable carrier is one or more selected from the group consisting of water, saline, aqueous glucose solution, aqueous pseudo-sugar solution, alcohol, glycol, ether, oil, fatty acid, fatty acid ester, glyceride, surfactant, suspending agent, and emulsifier.

10. The pharmaceutical composition for treating lung cancer according to claim 9, **characterized in that** the lung cancer is a lung cancer expressing ALK mutation and epidermal growth factor receptor (EGFR).
